# EUROPEAN PATENT SPECIFICATION

(11) **EP 0 298 654 B1**
(45) Date of publication and mention of the grant of the patent: **01.06.1994**
(21) Application number: 88305934.7
(22) Date of filing: 30.06.1988
(51) Int. Cl.: A61K 47/42, A61K 47/48

(54) **Use of antibody/antigen interactions to protect or modulate biological activity**
Verwendung von Antikörper-Antigen-Wechselwirkung zum Schutz oder Modulation der biologischen Aktivität
Utilisation des interactions entre anticorps et antigènes pour protéger ou moduler l'activité biologique

(30) Priority: 07.07.1987 US 71861; 18.04.1988 US 182530; 21.06.1988 US 205748
(43) Date of publication of application: 11.01.1989
(73) Proprietor: HYBRISENS LTD., Toronto Ontario M3J 1P3 (CA)
(72) Inventor: Shami, Ezekiel Y., Toronto Ontario (CA); Rothstein, Aser, Toronto Ontario (CA); Ramjeesingh, Mohabir, Mississaugu Ontario (CA)
(74) Representative: Arthur, Bryan Edward

(56) References cited:
- EP-A- 0 231 649
- EP-A- 0 251 001
- US-A- 4 704 692
- CHEMICAL ABSTRACTS, vol. 78, 1973, page 105, abstract no. 144674f, Columbus,Ohio, US; N. ZYK et al.: "Modification of L-asparaginase EC-2 by homologousantibodies", & BIOCHIM. BIOPHYS. ACTA. 1973,302(2), 420-8

## Description

This invention relates to the use of antibody/antigen interactions to protect biologically active entities against in vivo and in vitro inactivation.

Biologically active entities, such as enzymes, hormones, growth factors, antibodies and drugs, are used in a variety of medical and industrial applications in which their useful life may be shortened by inactivation. Such inactivation may result from physical, chemical or biological processes or conditions, or by self-destruction in the case of certain enzymes, occurring concurrently with the processes of the desired activity in a particular application; the inactivation may result from a combination of such inactivating processes. In some cases the inactivation occurs rapidly, necessitating frequent replacement of the active entity.

V. V. Mozhaev et al, Enzyme Microb. Technol., 1984, Vol. 6, page 50 et seq., review structure-stability relationships in proteins and existing approaches to stabilizing proteins. In Chemical & Eng'r News, September 30, 1985, page 19 et seq., there is a description of albumin/enzyme complexes that are resistant to proteolytic and heat inactivation, while U.S. patent No. 4,179,337 refers to polyethylene glycol/enzyme complexes which are also inactivation-resistant.

Biologically active entities are employed in a labelled form in different environments, for example, in immunological detection and diagnostic processes. The label may typically be a radioactive isotope label, enzyme label, fluorescent label or a label which can be determined photometrically. One limitation on the selection of the label is that it should not react with, and potentially inactivate, a site of desired biological activity of the biologically active entity.

Several solutions have been proposed to slow deterioration of activity in specific cases where a biologically active entity is subjected to inactivating conditions. However, no general approach has previously been formulated to counter the different inactivation processes with one type of agent.

In a publication by Zyk et al.,(Biochimica et Biophysica Acta, 302:420-28 (1973)): L-asparaginase/antibody complexes were examined to determine the nature and extent of changes in the stability of the enzyme when complexed with enzyme-specific antibody. The results showed that antibodies to L-asparaginase increased the stability of the enzyme with respect to thermal, proteolytic and pH-induced inactivation. In EPA 0 251 001 a preparation of pure IL-2 that is stable over long periods of time is disclosed. Stability is achieved by complexing IL-2 with a number of different serum proteins and evidenced by resistance to inactivation by freeze-thawing, lyophilization or pH change. The complex is the result of non-specific interactions between IL-2 and the serum proteins. EPA 0 231 649 discloses a storage-stable complex of albumin and another useful substance, such as vitamin E or a liquid fatty oil. The formation of the complex, a powder, does not result in a change in the quality of the useful substance included therein. The complex is the result of non-specific interactions between IL-2 and the serum proteins.

It is therefore an object of this invention to employ interactions between monoclonal or single-chain antibodies and biologically active antigens to effect a modulation, and particularly a prolongation, of their activity.
It is a further object of this invention to provide a biologically active entity stabilized against inactivation of a desired biological activity.

It is yet another object of the present invention to provide a mechanism for slow-release of a biologically active entity in order to provide sustained activity.

In accomplishing the foregoing objects, there has been provided, in accordance with one aspect of the present invention, a method of producing a biologically active complex characterised by an enhanced resistance to inactivation, comprising the steps of (A) binding a molecule which is biologically active with a monoclonal antibody or a single chain antibody that specifically binds said molecule to produce a biologically active complex; and (B) measuring a prolongation of biological activity, of said complex relative to the activity of said molecule alone when the complex is subjected to a condition that inactivates said molecule alone. In preferred embodiments, step (A) comprises exposing the biologically active molecule to a monoclonal antibody that recognizes the molecule. In another preferred embodiment, the antibody entity is an antibody fragment or a single-chain antibody.

In accordance with another aspect of the present invention, a complex has been provided that comprises (i) a molecule having a biological activity and (ii) a monoclonal or single-chain antibody entity recognizing that molecule, which complex displays a biological activity that is inactivation-resistant, relative to that of the free molecule. In a preferred embodiment, the molecule is an enzyme.

A method has also been provided, in accordance with still another aspect of the present invention, that comprises the steps of (1) providing a complex comprised of a molecule which is biologically active and an antibody entity as described above, said complex presenting at least one binding site for a labelling agent; (2) exposing the complex to the labelling agent such that the labelling agent is bound to the binding site; and then (3) effecting a disassociation of the complex to release the molecule carrying said labelling agent. In a preferred embodiment, the biologically active molecule is itself an antibody.

Other objects, features and advantages of the present invention will become apparent from the following detailed description. It should be understood, however, that the detailed description and the specific examples, while indicating preferred embodiments of the invention, are given by way of illustration.

The present invention is illustrated in the accompanying drawings, in which:
FIGURE 1 is a graph wherein the residual activity (in the presence of trypsin) of the biologically active entity, asparaginase, is plotted as a function of the increase in concentration of different antibody entities, or other proteins, employed to protect biological activity in accordance with the present invention.
FIGURE 2 is a graph showing the residual activity of asparaginase in the presence of trypsin, when the enzyme is protected with different combinations of monoclonal antibody in accordance with the present invention with time.

It has been discovered that a wide variety of biologically active molecules can be rendered inactivation-resistant by exploiting monoclonal or single-chain antibody/antigen interactions to protect vulnerable sites on such molecules from the harmful effects of any inactivation process, thereby dramatically slowing loss of biological activity.

To achieve a prolongation of biological activity in accordance with the present invention, a binding entity (as further defined below) is prepared that recognizes at least one site on a molecule ("biologically active entity"), that site being necessary to activity and normally subject to inactivation under certain conditions, such as disrupting temperature or pH or the presence of some agent like a proteolytic enzyme, an alcohol or an oxidant. A suitable binding entity in this context can be a monoclonal antibody which is raised by challenging the immune system of a standard laboratory animal with all or a portion of the biologically active entity. Alternatively, the binding entity can be a single-chain antibody which recognizes the biologically active entity. In any event, it is a routine matter to screen putative binding entities, pursuant to the present invention, to identify those having the requisite specificity, i.e., those that bind the biologically active entity in an inactivation-inhibitive manner without unduly lessening biological activity.

### (i) "Biologically Active Entity"

More specifically a biologically active entity suitable for the present invention can be any molecule that promotes or actively participates in a desired biological reaction and that has at least one first site responsible for, contributing to or participating in the desired biological reaction. Generally, a suitable biologically active entity will additionally have at least one second site which is essentially noncontributing to the desired biological reaction.

By "essentially noncontributing," it is intended that the at least one second site is not essential to the performance of the first site in the desired biological reaction. In particular, the second site may play a role in processes that result in inactivation of the biologically active entity, with respect to the desired biological activity. This inactivation may result from a physical, chemical or biological process or from a combination of such processes.

Biologically active entities which are used in the present invention can be enzymes, hormones, growth factors and antibodies, for example, as well as chemical species which effect biological change, such as drugs and medicines. Suitable biologically active entities include such enzymes as amylases, like α-amylase and β-amylase; glucoamylase, glucose isomerase, invertase; proteases like trypsin and subtilisin; pectinase, L-asparaginase, α-1,4-glucosidase, cholesteryl esterase, uricase, catalase, superoxide dismutase and glucose-6-phosphatase. Other biologically active entities are interferon, tissue plasminogen activator and muteins thereof, and such antibodies as CEA (carcinogen embryonic antigen), antibodies to HTLV, and antibodies to mouse IgG.

For enzymes, hormones and the like, there will typically be a plurality of the active first sites and, generally, a plurality of the second sites. Depending on the particular application, either the first or second sites, but not both, will participate in the antibody/antigen interaction. When the binding entity (see below) is an antibody entity, such sites will be epitopes to which the antibody entity will bind. In the case of drugs and medicines, the first site may be a chemical configuration or ligand responsible for the desired biological activity, and the second site may likewise be a chemical configuration or ligand.

Preferably, the first and second sites are spaced apart so that there is no stearic or other interference of the first site by the second site, after binding with the binding entity.

### (ii) "Binding Entity"

The binding entity is, in particular, a monoclonal antibody entity that "recognizes" (binds to) a specific portion or site of the biologically active entity. An antibody entity suitable for the present invention can be a monoclonal antibodie, or an amino-acid sequence which contains the variable region of an antibody. An antibody entity can also comprise hypervariable regions derived, respectively, from the heavy and light chains of an antibody, which chains could be linked:
- in their natural (in vivo) configuration, e.g., as in a Fab fragment,
- via chemical modification, using bifunctional linkers, to effect a crosslinking in vitro, or
- through a linking entity comprised of a variable-length peptide chain, thereby to provide a single-chain antibody or so-called "antigen binding protein," as disclosed, for example, in U.S. patent No. 4,704,692.

In yet another embodiment of the present invention, DNA encoding both the biologically active entity and the antibody entity could be integrated into a microorganism or animal cell, via recombinant DNA techniques, to effect a simultaneous synthesis of both entities, resulting in a complex of the two. Alternatively, the same approach could be employed to synthesize a new, covalently-integrated entity linking the biologically active molecule and antibody entity initially selected, either directly or via the linking entity, where complimentary sites on the two selected entities will form a complex.

An antibody entity within the present invention thus should be capable of binding to the second site(s) of the biologically active entity so as to prevent substantially or delay participation by the second site(s) in a process that leads to a deterioration of the desired biological activity to which at least one first site relates. The antibody entity may be formed in an immunological response, wherein the second site acts as an antigenic or antibody recognition site; this is not essential, however, as the antibody entity need only bind the second site so that it does not participate in an inactivation process. In addition, the antibody entity should not bind or interfere with the sites responsible for the desired biological activity to such a degree that the desired biological activity cannot serve a useful purpose.

Procedures are well-known for producing monoclonal antibodies, as well as fragments of antibodies, that will bind to a biologically active molecule. A suitable procedure can involve immunizing a rabbit or other standard laboratory animal with the biologically active entity, which may have been modified beforehand so as to "mask" the desired first sites and prevent the generation of inhibitory antibodies which recognize the first sites. For example, an antibody could be produced initially that binds a first site; then the complex of that antibody and the biologically active molecule would be used as the antigen to raise noninhibitory polyclonal sera. By means of conventional techniques, antisera taken from the animal can then be used as a source for antibody that binds the biologically active entity without destroying its activity, i.e., to produce antibody recognizing the second site(s), but not the active site, of the biologically active entity.

Conventional somatic-fusion procedures, as outlined, for example, by Kennett et al, Curr. Top. Microbiol. Immunol. 81: 77-91 (1978), can be employed to produce monoclonal antibodies suitable for use in the present invention. For example, mice can be immunized in a known manner with the biologically active entity, or a part of it. Spleen cells of the immunized mice are then removed and fused with an immortalizing cell line, such as murine myeloma line BALB/C NS-lAg4-1 (ATCC No. TIB18), according to the method of Kohler and Milstein, see, e.g., Nature 256: 495-97 (1975), and the resulting fusion products are screened for those that survive in culture and produce monoclonal antibody of the desired specificity. In this way, different hybridomas can be tested for production of monoclonal antibody that form, with the biologically active entity, a complex which displays prolonged activity (relative to the free molecule) under inactivating conditions.

In accordance with the present invention, a biologically active entity can be analyzed to identify specific sites which are to be bound by the binding entity. For example, fragments of the biologically active entity that do not include any active sites can be used as antigen to produce antibody which binds the biologically active entity without interfering with its activity.

When the biologically active entity is itself an antibody, its antigen binding site can be protected, pursuant to the present, using either antigen normally bound by the antibody or an anti-idiotypic antibody, i.e., an antibody that recognizes the antigen binding site.

### (iii) "Inactivation"

The biologically active entities employed in this invention may be inactivated as a result of physical, chemical or biological processes which may occur in the working environment of the entity. For example, disruptive increases or decreases in temperature, as well as oxidation may, result in inactivation. In the case in which the biologically active entity is an enzyme, inactivation may also result from enzymatic self-destruction.

A biologically active entity within the present invention can be employed in any environment in when the entity has previously been employed, as well as in environments where it has not been heretofore practical to use it because of short-lived activity. For example, use of enzymes as drugs or therapeutic agents is limited by their biodegradation or inactivation in the body; the present invention provides a means for overcoming this problem.

By the same token, an antibody which is used, according to the present invention, as a biologically active entity can be labelled or complexed with a desired drug, agent or other species without interfering with an active site of the antibody which is needed in the immunogenic or diagnostic process. Thus, antibodies are often chemically modified for the purpose of labelling. Most of these reactions are random and leave a certain percentage of the antibody inactive, due to the chemical modification of binding sites. This can be prevented, pursuant to the present invention, if before the chemical modification (labelling) the antibody is reacted with its antigen to form a complex according to the present invention (preferably, with antigen immobilized on a solid surface) so that the binding site is occupied and protected. Thereafter, when the labelling procedure is completed, the antibody/antigen complex can be dissociated, for example, with low pH buffer, and the labelled antibody collected and used.

Conversely, if an antigen is to be labelled, its antibody can immobilized, the antigen added, and labelling (and subsequent elution) carried out to remove the antibody.

Growth factors like interferon and erythropoietin have a very short half-life in serum, mainly due to enzymatic degradation. This problem is compounded when the drug is produced by engineered organisms within normal glycosylation is not effected and, as a consequence, carbohydrate residues found in the naturally-occurring molecule are missing. In the case of erythropoietin, these residues provide protection against enzymatic degradation. Specific antibody entities according to the present invention can provide similar protection, allowing for the use of the less expensive, genetically-engineered erythropoietin. Growth factors like epidermal growth factor (EGF), which are used to affect growth and proliferation of certain cell types, have an effectiveness that is diminished by degrading enzymes secreted by growing cells. Their efficacy can therefore be improved, in accordance with the present invention, by binding with an antibody entity.

Animal growth hormone can be used to increase body weight or milk production in farm animals. It is believed, however, that rapid inactivation of the injected hormone by proteolytic and other enzymes in vitro has made it necessary to use daily injections of the hormone, which is cumbersome. But protection of the growth hormone by binding the hormone with specific antibodies, in accordance with the invention, can protect the hormone from enzyme degradation without unduly reducing its potency. As a result, the effective hormone level can be maintained with lower doses and fewer injections.

In addition to protecting enzymes against inactivation by other enzymes, the present invention can be used protect an enzyme against self-degradation. For example, proteolytic enzyme, such as the subtilisin used in many detergents, can be protected against self-degradation by specific antibody entities pursuant to the present invention.

In accordance with the present invention, it is also possible to use a labelling entity which might otherwise be unsuitable as a result of side reactions affecting the desired site of activity. Thus, the desired site is initially bound to the binding entity to form a complex, thereafter the complex is labelled with the labelling agent by conventional procedures, the labelling entity being bound by the at least one second site. After the labelling entity is bound to the a second site, the binding entity is removed, by conventional procedures, from the labelled complex to provide the labelled biologically active entity in which the first site is free, the labelling agent being bound so that it is no longer available for reaction with the first site. By means of the present invention, a biologically active species can be complexed with the binding entity so as to assure the slow release over time of the species. In this way a single high dosage of the (complexed) species, which might otherwise be unacceptable because of toxicity or side effects, may be employed and frequent administrations (or higher dosages of a rapidly degraded species) consequently avoided.

The present invention is further described below by reference to the following, illustrative examples.

### Example 1. The Effect of Trypsin on Antibody-Protected and Unprotected Asparaginase

Monoclonal antibodies (MAbs) to L-asparaginase were prepared according to the method of Kohler and Milstein. Fifty micrograms of L-asparaginase suspended in phosphate buffer and Complete Freund's Adjuvant (1:1 volume ratio) were injected intraperitoneally into each of four BALB-C mice. A day 12 post-injection, a first boost was given i.p. in the form of 50 micrograms of the enzyme in phosphate buffer. On day 15, the mice were bled and their antibody titer was determined by the ELISA method. Those with the highest titer were given a second boost of the same constituency and, three days later, were sacrificed and their spleen cells were removed for use in a somatic fusion.

After 7 days, the supernatants from growing hybridomas were tested by the ELISA method for positive reaction against L-asparaginase. The most promising hybrids were cloned by the "limiting dilution method," as disclosed by Lefkovits and Waldmann, LIMITING DILUTION ANALYSIS OF CELLS IN THE IMMUNE SYSTEM (Cambridge Univ. Press 1979). Six clones of producing monoclonal antibodies were obtained and were labelled No. 12, No. 19, No. 29, No. 33, No. 34 and No. 35, respectively.

Five samples of enzyme-antibody complex were prepared as follows. To samples containing 25µl (0.05 units) of L-asparaginase were added antibody in an amount to provide ratios of 1, 2, 6, 10 and 20µg protein per unit of enzyme, respectively. Water was added to give a final sample volume of 500µl. Samples were prepared in this way with monoclonals No. 12, No. 29, No. 34 and No. 35, and with a bovine serum albumin (BSA), and were stored at 4°C overnight before testing, as described below.

In addition, 1.5 equivalents (3.75µg) of each enzyme-specific MAb were added to 0.5 unit (2.33µg) of L-asparaginase. In like fashion, samples of enzyme-antibody complex were prepared using four MAbS (Nos. 12, 29, 34 and 35) and three MAbs (Nos. 12, 29 and 34), respectively, in combination.

A Gilford "Response" spectrophotometer with temperature-controlled, 6-position, mm-cuvette holder was set at 37°C, and 50µl of each of the five samples prepared using one monoclonal antibody was added to separate cuvettes. Water (35µl) and trypsin (15 µl; 1.5 units) were added and the solutions incubated at 37°C for five minutes. At the end of this time the cuvettes were taken out and cooled in ice water. After spectrometer was readjusted to 25°C, the cuvettes were reinserted and allowed five minutes to equilibrate. Water (100µl; pH 9.0) and substrate (200µl) were added and mixed with a pasteur pipette. The conversion rate (decrease in absorbance/minute at 197nm) of L-asparagine to L-aspartic acid was determined for each sample.

In a separate experiment, 65µl (0.065 units) of each of the four multi-MAb samples were added to separate cuvettes of the spectrophotometer. Water (15µl), trypsin (20µl, 2 units) and substrate (200µl) were added, and the conversion rate of L-asparagine to L-aspartic acid was determined for each sample. A control sample, prepared by diluting 25µl of enzyme with water to a final volume of 650µl, was also run with and without the addition of trypsin.

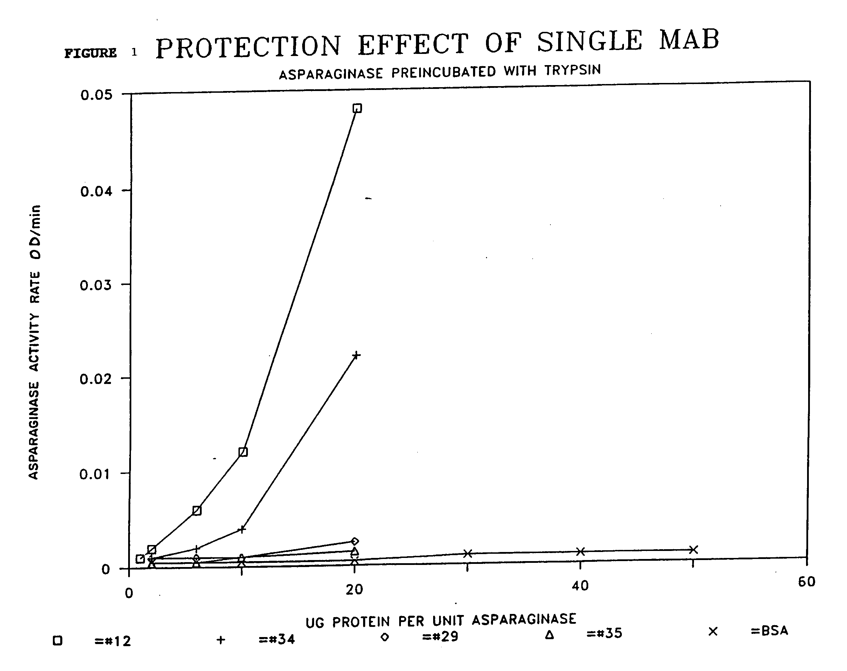

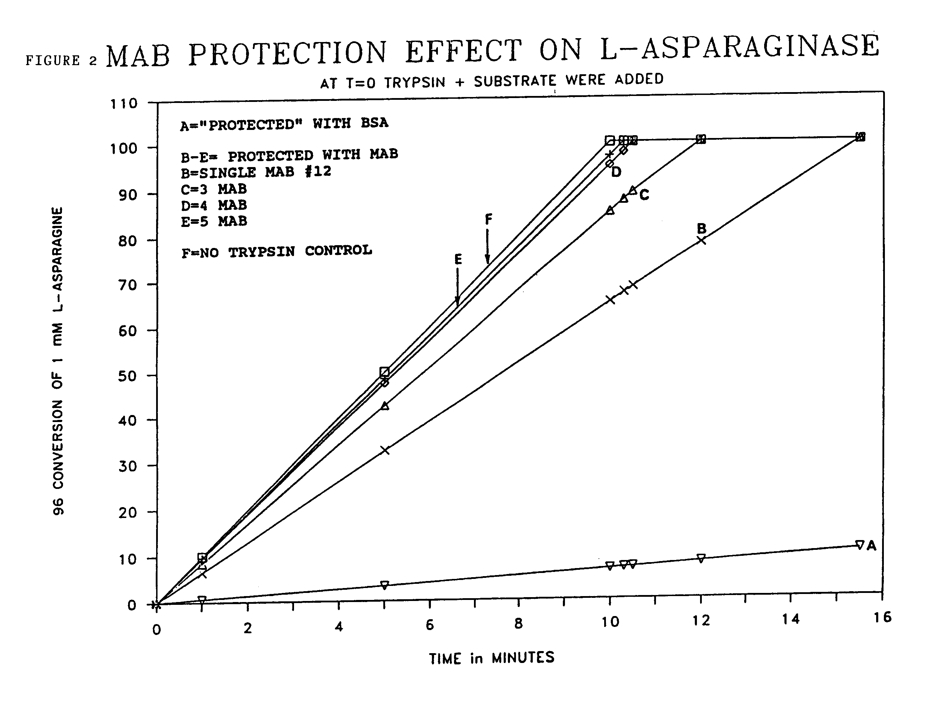

A plot of L-asparaginase residual activity versus antibody concentration (µg protein added per unit of L-asparaginase) for each of four single-MAb samples and for BSA-MAb sample is shown in Figure 1. The percent conversion of 1 mM of L-asparagine to L-aspartic acid versus time is plotted in Figure 2 for the multi-MAb samples. These results demonstrate that (1) the degree of protection varied from MAb to MAb, with No. 12 the most effective, and (2) an unrelated protein (BSA) provided virtually no protection. A significant level of protection was achieved with MAb No. 12 alone -- unprotected, trypsin-challenged enzyme lost over 90% of its activity under the same conditions -- but protection approximating that of the unchallenged control required the use of four or five monoclonal antibodies.

## Claims

1. A method of producing a biologically active complex characterised by an enhanced resistance to inactivation. comprising the steps of:
(A) binding a molecule which is biologically active with a monoclonal antibody or a single-chain antibody that specifically binds said molecule to produce a biologically active complex; and
(B) measuring a prolongation of biological activity of said complex relative to the activity of said molecule alone, when said complex is subjected to a condition that inactivates said molecule alone.

2. A method according to Claim 1, wherein said molecule is an enzyme.

3. A method according to Claim 1 or 2, wherein said condition is selected from disrupting temperature, the presence of a proteolytic enzyme, disrupting pH, the presence of an oxidizing agent, and the presence of an alcohol.

4. A method according to Claim 3, wherein said condition is the presence of a proteolytic enzyme.

5. A method according to Claim 3, wherein said condition is selected from disrupting pH, the presence of an oxidizing agent, and the presence of an alcohol.

6. A method according to any of Claims 1 to 5, wherein said molecule is subtilisin.

7. A method according to any of Claims 1 to 5, wherein said molecule is α-amylase.

8. A method according to any of Claims 1 to 5, wherein said molecule is L-asparaginase.

9. A method according to any of Claims 1 to 5, wherein said molecule is trypsin.

10. A method according to any of Claims 1 to 5, wherein said molecule is glucoamylase.

11. A method according to any of the preceding claims, wherein step (A) comprises binding a molecule which biologically active with a monoclonal antibody that specifically binds said molecule to produce a complex, wherein the biological activity of said complex is prolonged relative to the activity of said molecule alone, when said complex is subjected to a condition that inactivates said molecule alone.

12. A complex comprised of (i) a molecule having a biological activity and (ii) an antibody entity selected from the group consisting of a monoclonal antibody and a single-chain antibody that specifically binds said molecule, wherein the biological activity of said complex is prolonged relative to the biological activity of said molecule alone, when said complex is subjected to a condition that inactivates said molecule alone.

13. A complex according to Claim 12, wherein said molecule is an enzyme.

14. A complex according to Claim 13, wherein said molecule is subtilisin.

15. A complex according to Claim 13, wherein said molecule is α-amylase.

16. A complex according to Claim 13, wherein said molecule is L-asparaginase.

17. A complex according to Claim 13, wherein said molecule is trypsin.

18. A complex according to Claim 13, wherein said molecule is glucoamylase.

19. A complex according to any of Claims 12 to 18, wherein said complex is comprised of (i) a molecule having a biological activity and (ii) a monoclonal antibody recognizing said molecule.

20. A complex according to Claim 12, said complex being the product of a process comprising the steps of:
(A) binding said molecule and said antibody entity such that a molecule-antibody entity complex is formed; and
(B) measuring a preservation of said biological activity by said complex, relative to the activity of said molecule alone, when said complex is subjected to a condition that inactivates said molecule alone.

21. A complex according to Claim 20, wherein said condition is the presence of a proteolytic enzyme.

22. A complex according to Claim 20, wherein more than one monoclonal antibody or single-chain antibody is bound to said molecule.

23. A method labelling a biologically active molecule, comprising the steps of:
(A) providing a complex comprised of a molecule which is biologically active and an antibody according to claim 1, said molecule presenting at least one binding site for a labelling agent;
(B) exposing said complex to said labelling agent such that said labelling agent is bound to said binding site; and then
(C) effecting a disassociation of said complex to release said molecule bound to said labelling agent.

24. A method according to claim 23, wherein said molecule is an antibody.

25. A complex according to claim 12, being a recombinant simultaneously synthesized complex, comprising a biologically active entity covalently linked by a linking entity to a single chain antibody.

26. A continuous recombinant DNA sequence that codes for the recombinant complex of claim 25.

27. A recombinant complex according to claim 25, wherein the biologically active entity corresponds to an enzyme.

28. A recombinant complex according to claim 25, wherein the biologically active entity corresponds to a hormone.

29. A recombinant complex according to claim 25, wherein the biologically active entity corresponds to a growth factor.

30. A recombinant complex according to claim 25, wherein the biologically active entity corresponds to a drug.

31. A recombinant complex according to claim 25, wherein the biologically active entity corresponds to an antibody.

32. A recombinant complex according to claim 25, wherein said deactivating condition is selected from the group consisting of disrupting temperature, disrupting pH, the presence of a proteolytic enzyme, the presence of an oxidizing agent, and the presence of an alcohol.

## Patentansprüche

1. Verfahren zur Herstellung eines biologisch aktiven Komplexes, charakterisiert durch eine gesteigerte Resistenz gegenüber Desaktivierung, das folgende Schritte umfaßt:
(A) Verbinden eines Moleküls, das biologisch aktiv ist, mit einem monoklonalen Antikörper oder einem einfachkettigen Antikörper, der sich zur Bildung eines biologisch aktiven Komplexes spezifisch an das genannte Molekül anlagert; und
(B) Messen einer Verlängerung der biologischen Aktivität des genannten Komplexes relativ zu der Aktivität des genannten Moleküls allein, wenn der genannte Komplex einer Bedingung unterworfen wird, die das genannte Molekül allein desaktiviert.

2. Verfahren nach Anspruch 1, wobei das genannte Molekül ein Enzym ist.

3. Verfahren nach Anspruch 1 oder 2, wobei die genannte Bedingung ausgewählt ist aus disruptiver Temperatur, der Gegenwart eines proteolytischen Enzyms, disruptivem pH, der Gegenwart eines oxidierenden Agens und der Gegenwart eines Alkohols.

4. Verfahren nach Anspruch 3, wobei die genannte Bedingung die Gegenwart eines proteolytischen Enzyms ist.

5. Verfahren nach Anspruch 3, wobei die genannte Bedingung ausgewählt ist aus disruptivem pH, der Gegenwart eines oxidierenden Agens und der Gegenwart von Alkohol.

6. Verfahren nach einem der Ansprüche 1 bis 5, wobei das genannte Molekül Subtilisin ist.

7. Verfahren nach einem der Ansprüche 1 bis 5, wobei das genannte Molekül α-Amylase ist.

8. Verfahren nach einem der Ansprüche 1 bis 5, wobei das genannte Molekül L-Asparaginase ist.

9. Verfahren nach einem der Ansprüche 1 bis 5, wobei das genannte Molekül Trypsin ist.

10. Verfahren nach einem der Ansprüche 1 bis 5, wobei das genannte Molekül Glucoamylase ist.

11. Verfahren nach einem der vorhergehenden Ansprüche, wobei Schritt (A) das Verbinden eines Moleküls, das biologisch aktiv ist, mit einem monoklonalen Antikörper umfaßt, der sich an das genannte Molekül zur Bildung eines Komplexes spezifisch anlagert, wobei die biologische Aktivität des genannten Komplexes relativ zu der Aktivität des genannten Moleküls allein verlängert ist, wenn der genannte Komplex einer Bedingung unterworfen wird, die das genannte Molekül allein desaktiviert.

12. Komplex, der (i) ein Molekül mit einer biologischen Aktivität enthält und (ii) eine Antikörperentität, ausgewählt aus der Gruppe bestehend aus einem monoklonalen Antikörper und einem einfachkettigen Antikörper, der sich an das genannte Molekül spezifisch anlagert, wobei die biologische Aktivität des genannten Komplexes relativ zu der biologischen Aktivität des genannten Moleküls allein verlängert ist, wenn der genannte Komplex einer Bedingung unterworfen ist, die das genannte Molekül allein desaktiviert.

13. Komplex nach Anspruch 12, wobei das genannte Molekül ein Enzym ist.

14. Komplex nach Anspruch 13, wobei das genannte Molekül Subtilisin ist.

15. Komplex nach Anspruch 13, wobei das genannte Molekül α-Amylase ist.

16. Komplex nach Anspruch 13, wobei das genannte Molekül L-Asparaginase ist.

17. Komplex nach Anspruch 13, wobei das genannte Molekül Trypsin ist.

18. Komplex nach Anspruch 13, wobei das genannte Molekül Glucoamylase ist.

19. Komplex nach einem der Ansprüche 12 bis 18, wobei der genannte Komplex (i) ein Molekül mit einer biologischen Aktivität enthält und (ii) einen monoklonalen Antikörper, der das genannte Molekül erkennt.

20. Komplex nach Anspruch 12, wobei der genannte Komplex das Produkt eines Verfahrens ist, das folgende Schritte beinhaltet:
(A) Verbinden des genannten Moleküls und der genannten Antikörperentität, so daß ein Molekül-Antikörperentität-Komplex gebildet wird; und
(B) Messen der Aufrechterhaltung der genannten biologischen Aktivität durch den genannten Komplex relativ zu der Aktivität des genannten Moleküls allein, wenn der genannte Komplex einer Bedingung unterworfen wird, die das genannte Molekül allein desaktiviert.

21. Komplex nach Anspruch 20, wobei die genannte Bedingung die Gegenwart eines proteolytischen Enzyms ist.

22. Komplex nach Anspruch 20, wobei mehr als ein monoklonaler Antikörper oder einfachkettiger Antikörper an das genannte Molekül gebunden ist.

23. Verfahren zur Markierung eines biologisch aktiven Moleküls, das die Schritte beinhaltet:
(A) Bereitstellen eines Komplexes, der ein Molekül enthält, das biologisch aktiv ist und einen Antikörper gemäß Anspruch 1, wobei das genannte Molekül zumindest eine Bindungsposition für ein Markierungsagens aufweist;
(B) den genannten Komplex dem genannten Markierungsagens auszusetzen, so daß das genannte Markierungsagens sich an die genannte Bindungsposition anlagert; und dann
(C) Bewirken einer Dissoziation des genannten Komplexes, um das genannte, an das genannte Markierungsagens gebundene, Molekül freizusetzen.

24. Verfahren nach Anspruch 23, wobei das genannte Molekül ein Antikörper ist.

25. Komplex nach Anspruch 12, der ein rekombinanter, gleichzeitig synthetisierter Komplex ist, der eine biologisch aktive Entität enthält, die durch eine Bindungsentität kovalent an einen einfachkettigen Antikörper gebunden ist.

26. Eine kontinuierliche, rekombinante DNA-Sequenz, die den rekombinanten Komplex nach Anspruch 25 kodiert.

27. Rekombinanter Komplex nach Anspruch 25, wobei die biologisch aktive Entität einem Enzym entspricht.

28. Rekombinanter Komplex nach Anspruch 25, wobei die biologisch aktive Entität einem Hormon entspricht.

29. Rekombinanter Komplex nach Anspruch 25, wobei die biologisch aktive Entität einem Wachstumsfaktor entspricht.

30. Rekombinanter Komplex nach Anspruch 25, wobei die biologisch aktive Entität einer Droge entspricht.

31. Rekombinanter Komplex nach Anspruch 25, wobei die biologisch aktive Entität einem Antikörper entspricht.

32. Rekombinanter Komplex nach Anspruch 25, wobei die genannte desaktivierende Bedingung ausgewählt ist aus der Gruppe bestehend aus disruptiver Temperatur, disruptivem pH, der Gegenwart eines proteolytischen Enzyms, der Gegenwart eines oxidierenden Agens und der Gegenwart eines Alkohols.

## Revendications

1. Procédé de fabrication d'un complexe à activité biologique, caractérisé par une résistance accrue à l'inactivation, comprenant les étapes de :
(A) la liaison d'une molécule qui est active biologiquement , à un anticorps monoclonal ou à un anticorps à chaîne simple qui lie spécifiquement cette molécule pour produire un complexe à activité biologique ; et
(B) la mesure d'une prolongation de l'activité biologique de ce complexe par rapport à l'activité de cette molécule seule, lorsque ce complexe est soumis à une condition qui inactive cette molécule seule.

2. Procédé selon la revendication 1, dans lequel cette molécule est une enzyme.

3. "Procédé selon la revendication 1 ou 2, dans lequel cette condition est sélectionnée parmi la température de rupture, la présence d'une enzyme protéolytique, le pH de rupture, la présence d'un agent oxydant et la présence d'un alcool.

4. Procédé selon la revendication 3, dans lequel cette condition est la présence d'une enzyme protéolytique.

5. Procédé selon la revendication 3, dans lequel cette condition est sélectionnée parmi le pH de rupture, la présence d'un agent oxydant et la présence d'un alcool.

6. Procédé selon l'une quelconque des revendications 1 à 5, dans lequel cette molécule est la subtilisine.

7. Procédé selon l'une quelconque des revendications 1 à 5, dans lequel cette molécule est l'α-amylase.

8. Procédé selon l'une quelconque des revendications 1 à 5, dans lequel cette molécule est la L-asparaginase.

9. Procédé selon l'une quelconque des revendications 1 à 5, dans lequel cette molécule est la trypsine.

10. Procédé selon l'une quelconque des revendications 1 à 5, dans lequel cette molécule est la glucoamylase.

11. Procédé selon l'une quelconque des revendications précédentes, dans lequel l'étape (A) comprend la liaison d'une molécule qui est active biologiquement, à un anticorps monoclonal qui lie spécifiquement cette molécule pour produire un complexe, dans lequel l'activité biologique de ce complexe est prolongée par rapport à l'activité de la molécule seule, lorsque ce complexe est soumis à une condition qui inactive cette molécule seule.

12. Complexe constitué de (i) une molécule ayant une activité biologique et (ii) une entité anticorps sélectionnée parmi le groupe se composant d'un anticorps monoclonal et d'un anticorps à chaîne simple qui lie spécifiquement cette molécule, dans lequel l'activité biologique du complexe est prolongée par rapport à l'activité biologique de cette molécule seule, lorsque le complexe est soumis à une condition qui inactive cette molécule seule.

13. Complexe selon la revendication 12, dans lequel la molécule est une enzyme.

14. Complexe selon la revendication 13, dans lequel la molécule est une subtilisine.

15. Complexe selon la revendication 13, dans lequel la molécule est une α-amylase.

16. Complexe selon la revendication 13, dans lequel la molécule est une L-asparaginase.

17. Complexe selon la revendication 13, dans lequel la molécule est une trypsine.

18. Complexe selon la revendication 13, dans lequel la molécule est une glucoamylase.

19. Complexe selon l'une quelconque des revendications 12 à 18, dans lequel le complexe est constitué de (i) une molécule ayant une activité biologique et (ii) un anticorps monoclonal reconnaissant cette molécule.

20. Complexe selon la revendication 12, ce complexe étant le produit d'un processus comprenant les étapes de :
(A) liaison de la molécule et de l'entité anticorps de telle sorte qu'un complexe de molécule et entité anticorps est constitué; et
(B) mesure d'une conservation de l'activité biologique par ce complexe par rapport à l'activité de la molécule seule, lorsque le complexe est soumis à une condition qui inactive la molécule seule.

21. Complexe selon la revendication 20, dans lequel la condition est la présence d'une enzyme protéolytique.

22. Complexe selon la revendication 20, dans lequel plus d'un anticorps monoclonal ou anticorps à chaîne simple est lié à la molécule.

23. Procédé de marquage d'une molécule à activité biologique, comprenant les étapes de :
(A) production d'un complexe constitué d'une molécule qui est active biologiquement et d'un anticorps, selon la revendication 1, cette molécule présentant au moins un site de liaison pour un agent marqueur ;
(B) exposition de ce complexe à l'agent marqueur de telle sorte que l'agent marqueur est lié au site de liaison ; et ensuite
(C) réalisation d'une dissociation du complexe pour libérer la molécule liée à l'agent marqueur.

24. Procédé selon la revendication 23, dans lequel la molécule est un anticorps.

25. Complexe selon la revendication 12, étant un complexe recombinant synthétisé simultanément, comprenant une entité à activité biologique liée par covalence par une entité de liaison, à un anticorps à chaîne simple.

26. Séquence ADN recombinant continue qui code pour le complexe recombinant de la revendication 25.

27. Complexe recombinant selon la revendication 25, dans lequel l'entité à activité biologique correspond à une enzyme.

28. Complexe recombinant selon la revendication 25, dans lequel l'entité à activité biologique correspond à une hormone.

29. Complexe recombinant selon la revendication 25, dans lequel l'entité à activité biologique correspond à un facteur de croissance.

30. Complexe recombinant selon la revendication 25, dans lequel l'entité à activité biologique correspond à un produit pharmaceutique.

31. Complexe recombinant selon la revendication 25, dans lequel l'entité à activité biologique correspond à un anticorps.

32. Complexe recombinant selon la revendication 25, dans lequel la condition de désactivation est sélectionnée parmi le groupe se composant de la température de rupture, du pH de rupture, de la présence d'une enzyme protéolytique, la présence d'un agent oxydant et la présence d'un alcool.
